# EUROPEAN PATENT APPLICATION

(11) **EP 3 834 700 A1**
(43) Date of publication of application: **16.06.2021**
(21) Application number: 19214436.8
(22) Date of filing: 09.12.2019
(51) Int. Cl.: A61B 1/00, A61B 1/12, A61L 2/28

(54) **A MEDICAL APPARATUS, SYSTEM AND METHOD FOR INDICATING STERILIZATION**

(71) Applicant: Koninklijke Philips N.V., 5656 AG Eindhoven (NL)
(72) Inventor: HILGERS, Achim Rudolf, 5656 AE Eindhoven (NL); WEICHERT, Sven, 5656 AE Eindhoven (NL); VEENSTRA, Hugo, 5656 AE Eindhoven (NL); PELSSERS, Eduard Gerard Marie, 5656 AE Eindhoven (NL)
(74) Representative: Philips Intellectual Property & Standards

(57) **Abstract**

The invention provides a medical apparatus adapted to indicate whether it has been sterilized. The medical apparatus comprises a medical device and a dual-state sensor coupled to the medical device, wherein the dual-state sensor comprises two states. The first state of the dual-state sensor is a sterilized state based on the dual-state sensor being exposed to a sterilization process. The second state of the dual-state sensor is a used state based on the dual-state sensor being exposed to an external stimulus which indicates the medical device has been used. The invention allows a clinician to easily determine whether a medical apparatus has been used.

## Description

### FIELD OF THE INVENTION

The invention relates to systems and method for detecting sterilization of medical apparatus. In particular, the invention relates to indicating whether a medical apparatus has been used, and thus is not sterilized, and to counting how many times a medical apparatus has been sterilized.

### BACKGROUND OF THE INVENTION

Nosocomial infections (also known as hospital-acquired infections (HAI), hospital-associated infections, hospital infections) are infections that are not present in the patient at the time of admission to hospital but develop during the course of the stay in hospital. Amongst other infection routes "direct contact" is one of the major routes.

Direct contact between patients does not usually occur in healthcare facilities, but an infected healthcare worker can touch a patient and directly transmit a large number of microorganisms to a new host. The most frequent route of transmission, however, is indirect contact. The infected patient touches or will be in contact with medical devices during a medical examination or treatment and contaminates an object, an instrument, or a surface. Subsequent contact between that item and another patient is likely to contaminate the second individual who may then develop an infection.

Hospital acquired infections are a worldwide phenomenon. Patient care is provided in settings ranging from small healthcare clinics with basic facilities to large sophisticated highly equipped hospitals with state of the art technology. Despite progress in public health and hospital care, infections continue to develop in hospitalized patients and also in hospital staff. The World Health Organization (WHO) called HAIs a major cause of death and disability for patients. A survey on HAIs reveals that at any time, over 1.4 million people worldwide are suffering from infections acquired in treatment centers, with an estimated 80,000 deaths annually. The actual rates vary from 5% to 10% of all patients admitted to modern healthcare centers in the industrialized world to up to 25% in developing countries. The risk of healthcare associated infection in developing countries is 2 to 20 times higher than in developed countries.

Infection control in hospitals has always been a strong focus and nowadays becomes more and more regulated. Hospitals are eager to document that they do everything possible to prevent infections of their patients. Therefore means of tracking and recording medical devices, consumables and reusable devices are mandatory enablers.

An exemplary method of sterilizing a medical device is: the medical device is high-level disinfected via a machine cleaning program at certain conditions (e.g. 45 min at max. 90 °C) with a special detergent (details can be found in ISO 15883, the standard for an automated washer). The cleaned medical device will then secondly be sterilized for up to 20 minutes in an autoclave at up to 134 °C (details can be found in ISO 17665, the standard for steam sterilization) or other sterilizing techniques such as, for example, ethylene oxide (EtO) sterilization.

Manufacturers who provide autoclavable medical products, to prevent HAI, are eager to track the number of cleaning cycles a medical product has undergone, in case hospitals are sending in claims within the guarantee period and/or guaranteed number of allowed cleaning/disinfection cycles.

Therefore, there is a need to address the problem of uncertainty in whether a medical part (e.g. a consumable or reusable) is clean/disinfected and whether it can safely be (re-)used. There is also a need for a method to track the number of times that a device/part has been cleaned/disinfected.

### SUMMARY OF THE INVENTION

The invention is defined by the claims.

According to examples in accordance with an aspect of the invention, there is provided a medical apparatus adapted to indicate whether it has been sterilized, comprising:
a medical device;
a dual-state sensor coupled to the medical device, wherein the dual-state sensor comprises two states:
   a sterilized state based on the dual-state sensor being exposed to a sterilization process; and
   a used state based on the dual-state sensor being exposed to an external stimulus which indicates the medical device has been used.

The dual-state sensor is for indicating whether the medical device has been used. The medical device can be any conventional medical device used in the medical field, such as ECG sensors/cables, ultrasound probes, etc. The sensor may be placed in any position which may be easy to identify on the medical device. The sensor has two different states, sterilized or used. The used state is based on the medical device having been used, and is indicated by the sensor being triggered by an external stimulus (pushing, bending, electrical, etc.) applied to the sensor. Once the sensor is in the used state, it stays in this state until the medical device is sterilized. The sensor can then sense that it is being sterilized due to the, for example, high temperatures used during the sterilization process and change back into the sterilized state.

The dual-state sensor can therefore help a clinician determine whether they can use a medical device based on seeing whether the medical device has been used previously or not, as otherwise it would be difficult to determine whether it has been used.

The external stimulus may comprise one or more of:
an external mechanical force;
a current or voltage;
an electric field; and
a magnetic force.

The sensor can change to the used state based on an external stimulus. In an example, the external stimulus is a mechanical external force (pushing, bending, pulling, touching etc.) applied to the sensor. The sensor could be at the connector end of an ECG sensor cable, and once a mechanical external force is applied (by the clinician/nurse) the sensor then indicates that the sensor cable is being used. The sensor then stays in this state such that a clinician/nurse will not use the cables again until they have been sterilized.

In another example, a current or voltage could change the state of the sensor to a used state. The sensor could be electrically connected to the medical device, such that when the medical device is connected to a source of power, a current or voltage is applied to the sensor and the sensor thus changes to a used state. The sensor may then change to a sterilized state when it is sterilized (e.g. due to temperature change).

The dual-state sensor may comprise a switch electrically connected to the medical device and an indicator capacitor electrically connected to the switch.

The dual-state sensor may comprise a switch and an indicator capacitor. When the medical device is connected to a source of electrical power, the indicator capacitor may charge. A charged indicator capacitor thus indicates that the sensor is in a used state. The switch may then be configured such that during the sterilization process the switch closes, thus discharging the indicator capacitor. A discharged indicator capacitor thus indicates the sensor is in a sterilized state.

The switch may comprise one or more of: a temperature dependent mechanical switch; a bimetal switch; an electrical field dependent sensor; and a temperature dependent resistance. For example, a temperature dependent switch may close once it reaches a temperatures of 90 degrees Celsius (a common temperature during sterilization). This may be achieved with a bimetal switch. A temperature dependent resistance may also be used, wherein the resistance of the resistor changes with an increase in temperature (due to sterilization) thus changing the discharge rate of the indicator capacitor.

The dual-state sensor may comprise a shape memory alloy, SMA, temperature sensitive switch electrically connected to the medical device, wherein the SMA temperature sensitive switch is adapted to mechanically deform based on an external mechanical force and return to its original shape when exposed to a sterilization process.

For example, the SMA temperature sensitive switch may be closed whilst in the sterilized state. When the medical apparatus is first used after sterilization, the SMA temperature sensitive switch deforms (e.g. due to a connector part pushing the switch) and thus opening the switch, indicating the medical device has been used. The switch can be monitored to determine whether it is open or closed, thus indicating whether the medical device has been used.

The dual-state sensor may comprise:
a shape memory alloy, SMA, temperature sensitive switch adapted to return to its original shape when exposed to a temperature range for sterilization; and
a pin mechanically connected to the SMA temperature sensitive switch, wherein the pin is adapted to deform the SMA temperature sensitive switch when the medical apparatus is in use.

For example, the dual-state sensor could be part of electronic connector pin set-up. In a connector there are usually a plurality of pins. A pin may be extended further than the other pins and connected to an SMA temperature sensitive switch. When the connector is connected, the extended pin retracts, thus deforming the switch.

The dual-state sensor may further comprise an active electronic circuit electrically connected to the dual-state sensor, wherein the active electronic circuit is adapted to count the number of times the dual-state sensor has been in a used state.

The dual-state sensor may comprise a material adapted to change color based on being exposed to a sterilization process and return to an original color based on being exposed to an external mechanical force.

For example, the connector end of an ECG cable may comprise a button sensor made of a thermochromic material. The button sensor may be a green color when indicating the sterilized state. When the cable is connected to, for example, an ECG sensor, the clinician/nurse will push the button sensor and thus change the color of the button sensor to a red color. The button sensor will stay a red color (indicating it has been used) until the cable is sterilized. When the cable is being sterilized, the high temperatures of the sterilization process will change the color of the thermochromic material back to a green color.

The invention also provides a system for indicating whether a medical apparatus has been used or sterilized, the system comprising;
a medical apparatus coupled to a dual-state sensor, as defined above;
a patient monitor electrically connected to the medical apparatus, wherein the patient monitor is configured to detect the state of the dual-state sensor.

The patient monitor may be further configured to count how many times the dual-state sensor from the medical apparatus has been in a used state.

The invention also provides a method for indicating whether a medical apparatus has been used or sterilized, the method comprising:
indicating the state of a dual-state sensor on a medical apparatus, wherein the dual-state sensor comprises two states:
   a sterilized state based on the dual-state sensor being exposed to a sterilization process; and
   a used state based on the dual-state sensor being exposed to an external stimulus which indicates the medical device has been used.;
based on the dual-state sensor being in the sterilized state, indicating that the medical apparatus has been sterilized; and
based on the dual-state sensor being in the used state, indicating that the medical apparatus has not been sterilized.

The invention also provides a method for counting how many times a medical apparatus has been sterilized, the method comprising:
indicating whether a medical apparatus has been used or sterilized using the method defined above; and
based on the medical apparatus having been sterilized, increasing a count by one, wherein the count indicates the number of times the medical apparatus has been sterilized.

The method may further comprise:
displaying the count; and
displaying the state of the sensor.

The method for counting how many times a medical apparatus has been sterilized may further comprise:
determining a unique identifying number for a medical apparatus;
obtaining a count associated with the unique identifying number; and
based on the medical apparatus having been sterilized, increasing the count by one.

The invention also provides a computer program comprising code means for implementing any of the methods mentioned above when said program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figures 1A and 1B show a cable with a dual-state sensor;
Figures 2A and 2B show medical devices with a dual-state sensor connected to a patient monitor;
Figure 3 shows a first example of a circuit used as a dual-state sensor;
Figure 4 shows a second example of a circuit used as a dual-state sensor;
Figures 5A, 5B, 5C and 5D show a male connector with a dual-state switch made from shape memory alloy and female connector;
Figures 6A and 6B show a close up of how a pin interacts with a shape memory alloy;
Figures 7A and 7B show a connector with a dual-state sensor made from a shape memory alloy and a socket.
Figures 8A and 8B show a connector with a dual-state sensor which changes color.
Figure 9 shows the processing method used to indicate whether a medical apparatus has been used.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a medical apparatus adapted to indicate whether it has been used (and is therefore not sterile) or sterilized (and not subsequently used). The medical apparatus comprises a medical device and a dual-state sensor coupled to the medical device, wherein the dual-state sensor comprises two states. The first state of the dual-state sensor is a sterilized state based on the dual-state sensor being exposed to a sterilization process. The second state of the dual-state sensor is a used state based on the dual-state sensor being exposed to an external stimulus which indicates the medical device has been used. The invention allows a clinician to easily determine whether a medical apparatus has been used.

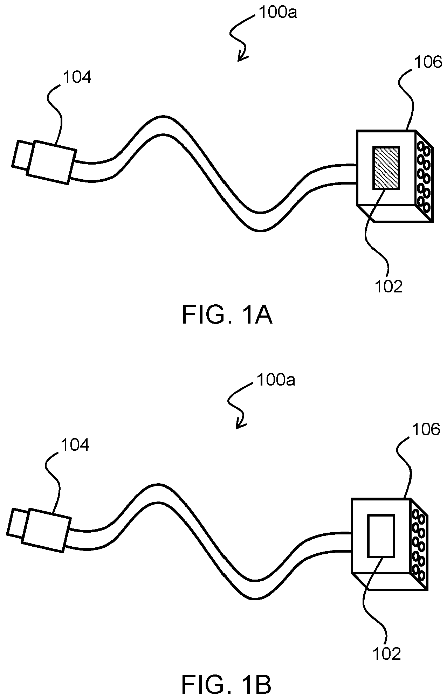

Figures 1A and 1B show a cable 100a with a dual-state sensor 102. The cable 100a is shown with an ECG measurement dongle 106 (which is one example of a medical device) and a digital connector 104 (e.g. USB). The dual-state sensor 102 is coupled to the ECG measurement dongle 106 which indicates whether the cable 100a has been used or whether it is sterilized. The combination of the medical device and the dual-state sensor may be considered to be a medical apparatus. The two states possible for the dual-state sensor 102 are: a sterilized state, to indicate the cable 100a has been sterilized, and a used state, to indicate the cable 100a has been used. Figure 1A shows the dual-state sensor 102 in a sterilized state and Figure 1B shows the dual-state sensor 102 in a used state.

By distinguishing between a sterilized state and a used state, it is possible for a clinician to easily determine whether they can use the cable 100a (if it is sterilized) or not (if it has been used) without any other knowledge of the past history of the cable 100a.

For example, the dual-state sensor 102 could indicate that the cable 100a has been used when the USB connector 104 is plugged into a patient monitor, thus triggering the dual-state sensor 102 to be in the used state. Alternatively, the dual-state sensor 102 could indicate the cable 100a has been used when an external mechanical force is applied by a clinician (e.g. the dual-state sensor 102 is pushed/bent when a clinician holds the ECG measurement dongle 106 to connect it to a set of ECG sensors). It may also be possible for the dual-state sensor 102 to indicate the cable 100a has been used when the ECG measurement dongle 106 is connected to a set of ECG sensors. In this case, the physical connection between the ECG measurement dongle 106 and the ECG sensors would trigger the dual-state sensor 102 to be in the used state.

Thus, preferably the normal use of the medical device triggers the dual-state sensor to trigger, as a result of the forces or other external stimulus to which the medical device is subjected in normal use. Alternatively, a clinician may need to perform a step as part of the standardized use of the medical device thereby to provide an appropriate external stimulus.

Although the description above is based on an ECG cable 100a, a dual-state sensor 102 may be coupled to a variety of re-usable medical devices 100.

It may also be possible for non-physical stimuli to trigger the dual-state sensor 102 to change state. For example, if a medical device 100 is connected to a patient monitor by a wireless connection (e.g. Bluetooth, Wi-Fi), the dual-state sensor 102 may be triggered by the wireless connection and thus change to the used state. The dual-state sensor 102 may also be triggered by an external electromagnetic field (e.g. radio waves, X-rays, infrared, or light of any specific wavelength), wherein the use of the medical device 100 is accompanies by the medical device 100 receiving, for example, radio waves. Alternatively, the dual-state sensor 100 may change to a used state based on specific acoustic signals (e.g. ultrasound, noise).

There are various manual and automated options to realize a dual-state sensor 102. Each option has pro and cons, and depending on the use case, a different option may be preferred.

The dual-state sensor 102 is designed to return to a sterilized state when the medical device 100 (e.g. the ECG cable) is sterilized. Sterilization usually involves high temperatures (typically above 90 degrees Celsius). Therefore, the dual-state sensor 102 reaching a temperature of over 90 degrees Celsius may trigger the dual-state sensor 102 to change to the sterilized state, and thus allowing a clinician to determine that the medical device 100 has undergone a disinfecting/sterilizing cycle and can be used in the future.

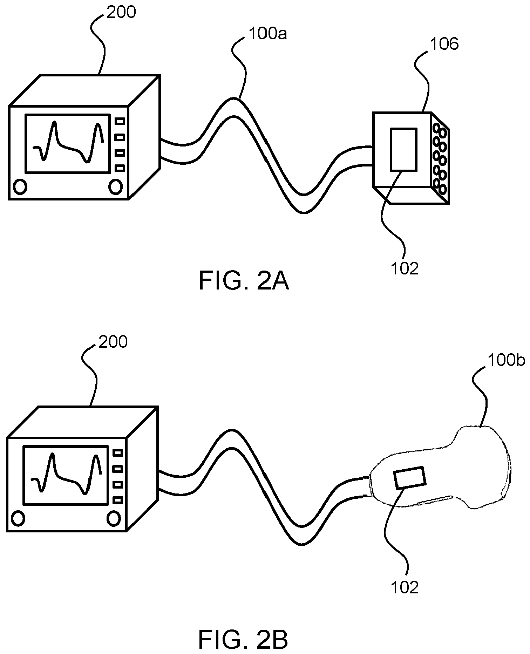

Figures 2A and 2B show medical devices 100 with a dual-state sensor 102 connected to a patient monitor 200. As discussed, a patient monitor 200 may be used to trigger the dual-state sensor 102 to change to a used state. Figure 2A shows an ECG measurement dongle 106 connected via a cable 100a to a patient monitor 200. The power provided by the patient monitor 200 may trigger the dual-state sensor 102 to change to a used state. The power provided by the patient monitor 200 may be AC or DC power.

Alternatively, the physical connection between the patient monitor 200 and the cable 100a may trigger the dual-state sensor 102 to change to the used state. In either case, the patient monitor 200 may be configured to display the state of the dual-state sensor 102 (the state of the sensor 102 directly before being plugged in). The displayed state of the dual-state senor 102 may help a clinician decide whether they can use a medical device 100.

Figure 2B shows an ultrasound probe 100b connected to a patient monitor 200. The patient monitor 200 may be configured to process ultrasound data from the ultrasound probe 100b. The ultrasound probe 100b is coupled to a dual-state sensor 102. As discussed above, the dual-state sensor 102 may change state based on electrical power, mechanical forces etc. However, in this case, the dual-state sensor 102 may change state based on receiving ultrasound signals, such that when the ultrasound probe 100b is turned on (and thus transmitting and receiving ultrasound signals), the dual-state sensor 102 changes state to the used state.

However, the use of a dual-state sensor 102 is not limited to ECG measurement dongles 106 and ultrasound probes 100b. For example, a dual-state sensor 102 may be coupled to a thermal probe, a heart rate sensor or any sensor which may be connected to a patient monitor 200 and/or a computer.

The patient monitor 200 may also be used to count how many times a medical device 100 has been used. For example, a clinician could input which medical device 100 it is using to the patient monitor 200, and the patient monitor 200 could count how many times the particular medical device 100 has been used. Alternatively, the patient monitor 200 may receive a unique identifying number for each medical device 100 and increase the count associated with the unique identifying number when the particular medical device 100 is connected to the patient monitor 200. The patient monitor 200 may be connected to other patient monitors such that when the particular medical device 100 is used on a different patient monitor, the count is increased on all patient monitors. Alternatively the count for each medical device 100 may be stored in a centralized system which is connected to all the patient monitors.

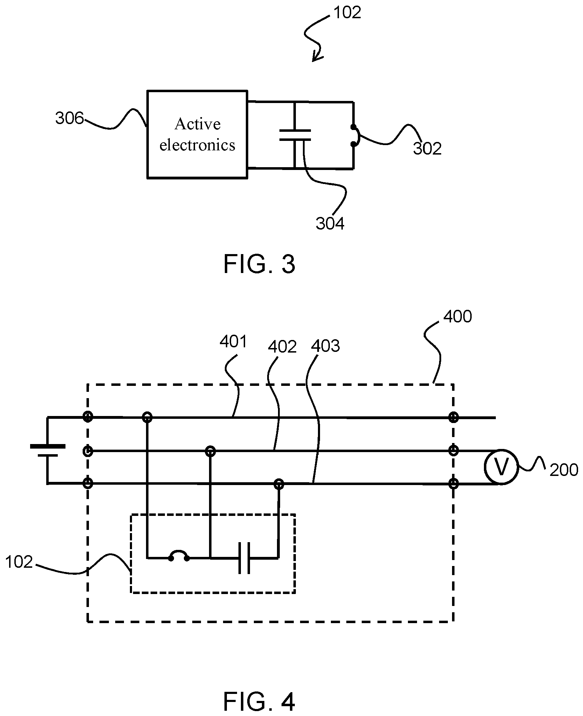

Figure 3 shows a first example of a circuit used as a dual-state sensor 102. An electronic circuit 306 (operated by the power supply provided by the digital connector 104) may charge an indicator capacitor 304 with capacitance C(indicator) while the digital connector 104 is connected to the patient monitor (PM) 200. For this, at least one additional connection between the dongle and the leadset is required, wherein the additional connection is dedicated to interface with the capacitor 304. One side of the capacitor 304 may be connected to the shield connection of the leadset, which is already present at the leadset connector.

The charge will remain stored in the capacitor 304 as long as no high temperature (the autoclave) cycle will be undergone. If a certain temperature is reached during the sterilizing process (e.g. the required 134 °C) a mechanical switch 302 (e.g. bimetal switch) will short the capacitor 304, which will be discharged accordingly. When the digital connector 104 is connected to the patient monitor 200, the active electronics circuit 306 may check the charge of said indicator capacity 304. If non-zero, the patient monitor 200 can determine that this leadset was used and may not be clean/sterile anymore. However, if the charge is zero, the patient monitor 200 identifies this configuration as clean/sterile and marks the medical device 100 as "used" by charging the capacitor 304. As long as the connection is not interrupted (disconnecting the digital connector 104 from the patient monitor 200) there is no need to re-check the status again.

As a digital connector 104 may allow for exchanging data, the number of sterilizing cycles may be derived from how often an indicator capacitor 304 has been recharged. When an active electronic 306 is used, unique identifying numbers (for identifying a leadset) may be stored. The information about the charging status of the indicator capacitor 304 may be, for example, sent via the ID bus or modulated on top of the dc-supply or using either the Tx or Rx pairs (transmitting and receiving pair of lines). First, after attaching the digital connector to the patient monitor 200, the charging status will be checked and corresponding data will be exchanged.

Instead of using a mechanical switch 302, such as a bimetal switch to discharge the indicator capacitor, a temperature dependent resistance (NTC) or a series circuit consisting of a bimetal and a NTC could be used for identifying how often a device/part 100 has undergone the high temperature process of the sterilizing procedure. Thus, each time the temperature threshold has been reached, the capacitor 304 will be discharged via the NTC/bimetal switch according to a pre-defined time constant τ (τ = R(NTC) x C(indicator)). In this way, during the autoclave process the capacitor 304 loses some charge (corresponding to the charged voltage amplitude, Q = C(indicator) x V(charge)). Accordingly, the charged voltage (amplitude) is an indicator for how often the medical device 100 has been sterilized. Once the charge/voltage is (close to) zero, the medical device 100 may be thrown away or the patient monitor 200 could charge the capacitor 304 again. In such a configuration, re-charging the capacitor 304 by the patient monitor 200 may be not beneficial.

As explained above, the digital connector 104 in this example is an interface between the patient monitor 200 and a smart measurement dongle (such as for ECG measurement). The digital connector interface between the monitor and dongle provides power to the dongle and allows for bidirectional communication. An ECG leadset is connected to the ECG measurement dongle.

At least one dc power supply is available to the dongle when the digital connector 104 is connected to the patient monitor 200, such that the active electronic circuit 306 may be supplied with power. The dual-state sensor 102 may be incorporated in either the digital connector 104 and/or the smart dongle (which is connected to the digital connector's 104 opposite end).

The active electronic circuit 306 may include an electronic counter. The counter is incremented each time (by one) the digital connector (and/or smart dongle) is connected to the monitor 200. To count the frequency of autoclave cycles a certain condition needs to be checked. This condition could be a "function reset" during the autoclave process and a "function set" during the first time the digital connector 104 (and/or smart dongle) is connected to the patient monitor 200. If an active electronic circuit 306 is used, a unique identifying number (for identifying a digital connector and/or dongle) may be stored.

Figure 4 shows a second example of a circuit used as a dual-state sensor 102. A dual-state sensor 102 may also be part of a passive leadset 400 which may be connected to, for example, a trunk cable or a dongle connector. For example, a dual-state sensor 102 may be part of a 5 lead Electrocardiography (ECG) cable. A patient monitor 200 may be connected with a digital connector/dongle 104 to the trunk and the (5) leads. The leads then connect to the corresponding electrodes. Although each electrode has a separate signal line (wire) at least one common ground electrode may be shared (functioning as a shielding electrode).

The dual-state sensor 102 shown in Figure 4 can be implemented in any leadset 400 with at least three channels/lines 401 402 403. For example, a small (to avoid disturbing ECG signals during normal ECG measurements) capacitor between wire two 402 and wire three 403 of a leadset 400 and a bimetal switch between wire one 401 and wire two 402 may be implemented, for example they may be conveniently located in the socket/connector of the leadset 400. During the autoclave process, a dc voltage may be applied between wire one 401 and wire three 403 (e.g. provided via a connector in the autoclave). If the threshold voltage of the bimetal switch is reached, the bimetal switch closes and charges the capacitor. After the autoclave process, when the leadset 400 is connected to the patient monitor 200, the patient monitor 200 checks the charge (voltage) of the capacitor between wire two 402 and wire three 403. If it is charged, the leadset 400 can be considered to be clean/disinfected/sterile (and the patient monitor discharges the capacitor to mark it as "used"). If the capacitor is already discharged, the patient monitor 200 identifies this leadset 400 as not clean/used.

In an example, the capacitor may be charged by the high temperatures of the sterilization process. For example, a piezoelectric material may be connected to the capacitor such that the capacitor is charged when the piezoelectric material is at a high temperature (e.g. 90 degrees Celsius for sterilization). Alternatively, a light sensor may be used to charge the capacitor during the sterilization process. Sterilization/disinfection may involve bright lights (e.g. ultraviolet germicidal irradiation) which can cause a light sensor to create a voltage potential and charge the capacitor. The leadset 400 may, in this case, be (partially) optically transparent to certain wavelengths, wherein the certain wavelengths depend on the wavelengths used during sterilization which the light sensor is adapted to absorb.

Due the fact that the ("auto-orange") leadset needs separate shield connections (this is needed to implement electrosurgical, ESU, filtering) which uses a series resistor inside the shield for each electrode, this resistor may be placed inside the dongle. A capacitor could be placed inside the leadset, connected between two shield connections of the leadset. This would avoid a reduction in impedance between the electrode(s) and the shield.

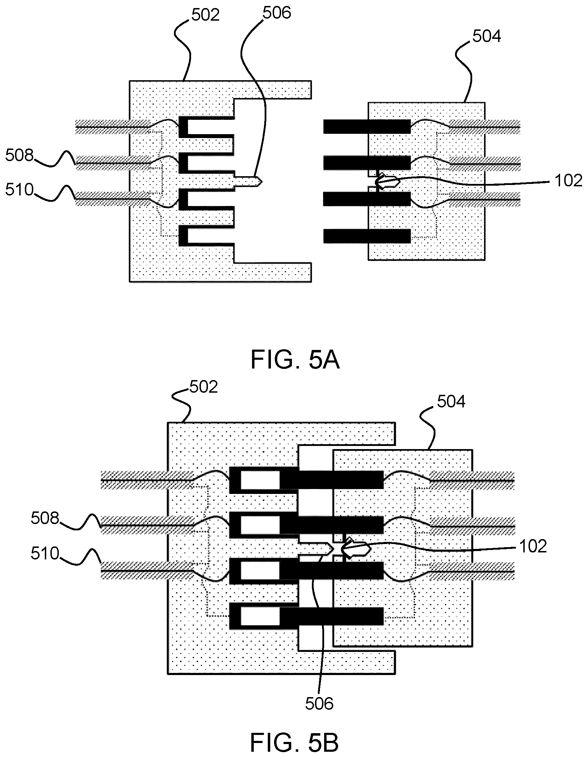

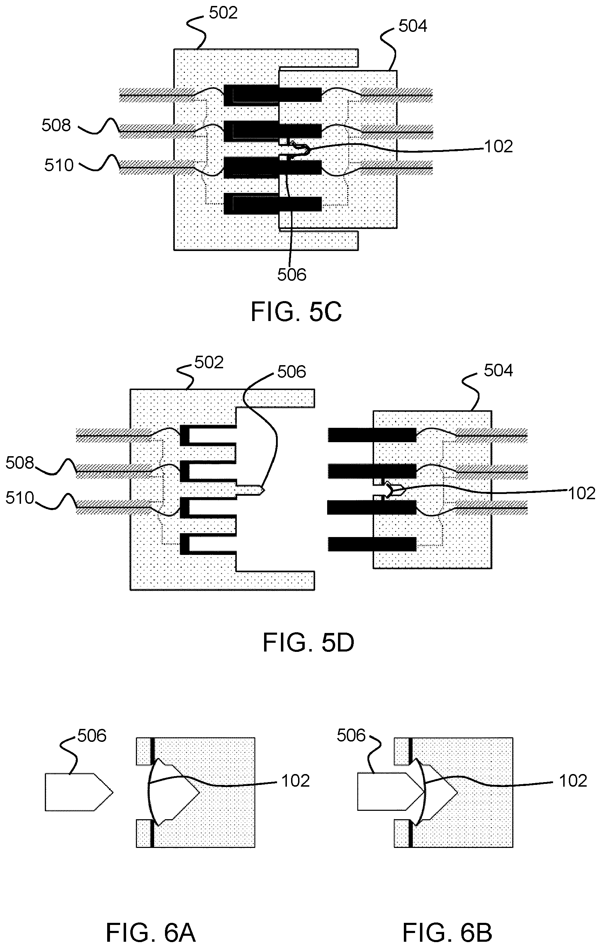

Figures 5A, 5B, 5C and 5D show a male connector 504 with a dual-state switch 102 made from shape memory alloy and female connector 502. A shape memory alloy (SMA) 102 with a particular shape can be deformed mechanically and subsequently retuned to its original shape when the SMA 102 is heated. This effect can be used to design a dual-state sensor 102 which acts as an electrical switch that closes when heated and opens when a mechanical actuation is applied. When the mechanical actuation is ceased, the switch remains open.

When the SMA 102 is deformed, a reconnection of the male connector 504 and female connector 502 will not lead to an electrical connection between wire two 508 and wire three 510. Only after sterilization in an autoclave, the electrical connection can be reinstalled. This is a very useful feature in the patient monitoring, warranting safe re-use, as described below:
State (1) - male connector 504 and female connector 502 are disconnected, the SMA is not deformed, as shown in Figure 5A.
State (2) - male connector 504 and female connector 502 are partially closed, the SMA 102 is not deformed, as shown in Figure 5B.
State (3) - male connector 504 and female connector 502 are fully closed, the SMA 102 is deformed, as shown in Figure 5C.
State (4) - male connector 504 and female connector 502 are disconnected, the SMA 102 is deformed, as shown in figure 5D.

Initially in state (1), the parts of the connector are separated. When an operator (e.g. nurse) pushes the female part 502 and male part 504 of the connector together, an electrical connection between wire two 508 and wire three 510, as depicted in state (2), is temporarily noted by the patient monitor 200 (which is connected to the female part 502 of the connector) and thereby the patient monitor 200 recognizes the medical device 100 (connected to the male part 504 of the connector) as clean/sterilized. When in state (3) the female part 502 and male part 504 of the connector are fully closed and the electrical wires 2 and 3 are again disconnected because the pin 506 on the female part 502 has deformed the SMA 102. The patient monitor 200 recognizes this disconnection and the patient monitor starts to record signals originating from the medical device 100. After measurements, the operator disconnects the female part 502 and male part 504 of the connector as depicted in Figure 5D for state (4). If subsequently the operator does not autoclave the medical device 100, including the male part 504 of the connector, and would try to reuse the medical device 100 by reconnecting it to the female part 502, no electrical connection between wire two 508 and wire three 510 will be recorded by the patient monitor 200 and the patient monitor 200 recognizes that the medical device 100 has already been used (and that it is not autoclaved). Consequently the patient monitor 200 may issue an alarm that the medical device 100 should be autoclaved. If the medical device 100, including male part 504 of the connector, is autoclaved, the SMA 102 returns to its original shape as depicted in Figure 5A for state (1) and when the operator reconnects the male part 504 and female part 502 of the connector, the electrical connection between wire two 508 and wire three 510 is again enabled and the patient monitor 200 will allow reuse of the medical device 100.

Figures 6A and 6B show a close up of how a pin 506 interacts with a shape memory alloy 102. The pin 506 pushes the SMA 102 out of shape, thus disconnecting the small connectors on either side of the SMA 102. The small connectors are connected to wire two 508 and wire three 510 when the male part 504 and female part 502 are not closed, such that, when the SMA 102 is not deformed, there is an electrical connection between wire two 508 and wire three 510. Figure 6A shows the SMA 102 when not deformed. When the male part 504 and female part 502 are fully connected, the pin 506 deforms the SMA 102. Figure 6B shows the pin 506 deforming the SMA 102, and thus disconnecting the wire two 508 and wire three 510.

As explained above, when the patient monitor 200 records the electrical connection between wire two 508 and wire three 510, an electronic counter within the patient monitor 200 may be incremented with one. Every time a medical device 100 is reused and recognized as being autoclaved, the number in the counter is increased by one. Typically the medical device 100 (including the male part 504 of the connector) can be used one hundred times and when the counter has reached one hundred, the patient monitor 200 will issue a warning that the medical device 100 cannot be used anymore after the next measurement and should be discarded. If the operator autoclaves the medical device 100 again and tries to re-use it, the patient monitor 200 may issue an alarm of invalid use and/or could even refuse to use the medical device 100. When the operator uses a new device, the operator may reset the patient monitor 200 counter and the new device can be used. If the operator forgets to reset the counter, the patient monitor 200 may issue another alarm of invalid use, thus reminding the operator to reset the counter.

One safety issue has to be considered still: if an operator uses for instance two equal medical devices intermittently, the counter would reach the maximum before the actual discarding has to be carried out. Two solutions are possible for this issue:
(1) In the instructions for use it is stated that only a new medical device should be used after having reached the maximum re-use of a previous medical device. However this will require fast autoclavation.
(2) The SMA's 102 as used in various medical devices have a different electrical resistance which can be noted by the patient monitor 200. For instance, when using 10 different electronic counter circuits, the patient monitor 200 can record the use of up to ten different medical devices simultaneously. Solution (2) has two additional advantages: if the operator forgets to reset the patient monitor 200 counter, the patient monitor 200 still knows that it is a new device and will use a subsequent counter to record the number of autoclave sterilizations; and no need for fast autoclave sterilization to enable immediate reuse.

There are various types of SMA's, including SMA's that operate in the range of 100 to 400 degrees and are suitable for switching when autoclaved, but do not switch when remaining on room temperature or being cleaned at, for example, 90 degrees Celsius.

The advantages of using an SMA switch as the dual-state sensor 102 are:
(1) There are no electrical components required that need to be autoclaved.
(2) The operator receives an automatic notification if the reset of the counter is forgotten by the operator.
(3) No additional conducting pins are required in the socket and connector.
(4) Each medical device 100 can have a "fingerprint" (e.g. different electrical resistance of SMA). By fingerprinting a particular device, it can be identified by the patient monitor 200, allowing for unambiguous counting of the number of autoclave sterilizations. This is suitable where additional interference in workflow cannot be tolerated or is highly undesirable, for instance in the intensive care unit or general ward. The disadvantage is that in the patient monitor 200 an adaptation is required for the software to enable measurement on the moment the socket is pushed into the connector. However, since voltages are already measured between the pins, no hardware modifications are required to the patient monitor 200.

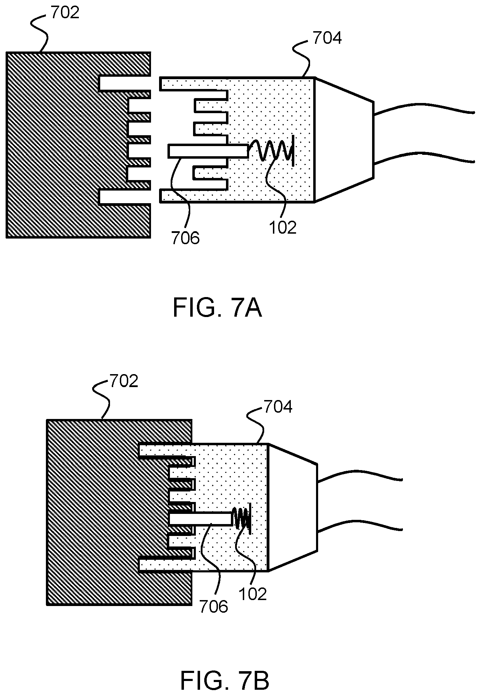

Figures 7A and 7B show a connector 704 with a dual-state sensor 102 made from a shape memory alloy and a socket. In this example, a pin 706 of a connector 704 protrudes further than the other connector pins when in a sterilized state. A socket 702 may be connected to a monitoring device 200 (e.g. a patient monitor). The monitoring device 200 can notice the protruding pin 706 being in contact with the socket 702 before the other pins. The protruding pin 706 will be pushed inwards when the connector 704 is fully inserted in the socket 702. The protruding pin 706 is connected to a dual-state sensor 102 made from a shape memory alloy (e.g. a shape memory alloy spring). When the connector 704 is fully connected to the socket 702, the spring 102 is deformed by the protruding pin 706 being pushed back. Figure 7B shows the connector 704 fully connected to the socket 702 and the protruding pin 706 fully pushed back. Once the connector 704 is disconnected, the protruding pin 706 stays pushed back.

The spring 102 behind the pin 706 will push the pin 706 outwards again during the high level disinfection process (e.g. due to the high temperatures of the cleaning/sterilization process). In case the connector 704 is connected again, without running it through the cleaning/sterilization process, the monitoring device 200 will not sense the initial contact with the protruding pin 706, thus will determine the connector 704 is not clean.

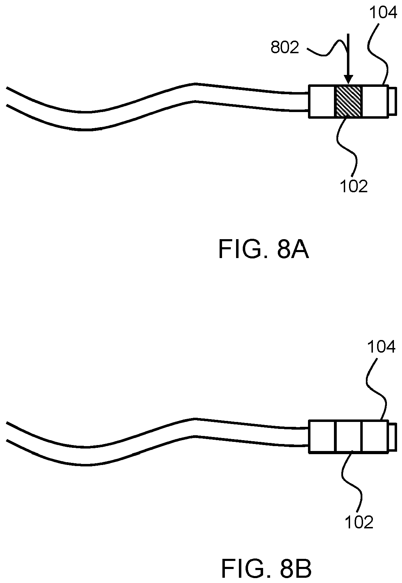

Figures 8A and 8B show a digital connector 104 with a dual-state sensor 102 which changes color. In this example, part of a digital connector 104 (or a cable) may be made from a material, which changes color (thermo-chromic materials) and can go back to an original color after applying a force 802 to it. For example, a green color indicates that it is clean and a red color indicates that it has been used. Before use, a force 802 needs to be applied (bending, pushing) to change the color to its original color, which indicates that the digital connector 104 is used. The color change of the part 104 will be reversed through the high temperature during the high level disinfection process.

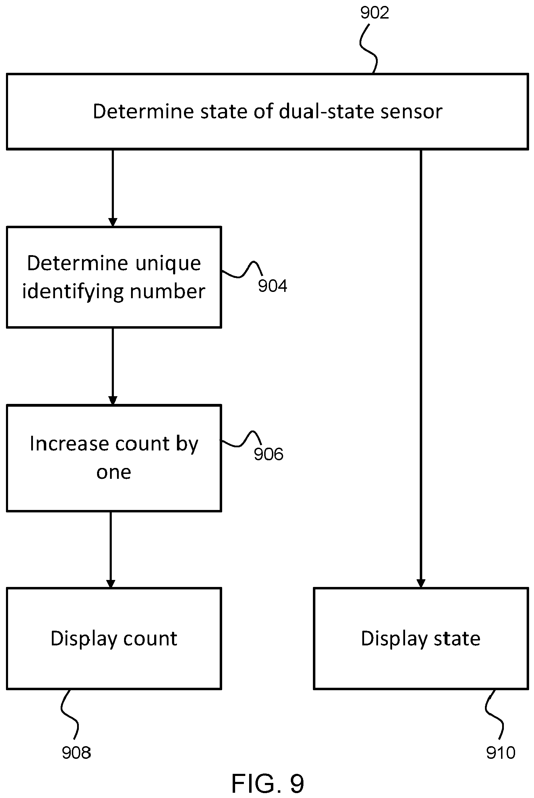

Figure 9 shows the processing method used to indicate whether a medical apparatus has been used. First, in step 902, the state of a dual-state sensor is determined, wherein the dual-state sensor is coupled to the medical device. The two states of the dual-state sensor are: a sterilized state based on the medical device being sterilized/clean, and a used state based on the medical device having been used (and thus not sterilized/clean). Optionally, in step 904, if the state of the dual-state sensor is "sterilized", a unique identifying number is determined for the medical device and a count associated with the unique identifying number is increased by one, in step 906. The count (of how many times the medical device has been sterilized) is displayed, in step 908, and the state of the dual-state sensor is also displayed, in step 910.

The skilled person would be readily capable of developing a processor for carrying out any herein described method. Thus, each step of a flow chart may represent a different action performed by a processor, and may be performed by a respective module of the processing processor.

The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A medical apparatus adapted to indicate whether it has been sterilized, comprising:
a medical device (100);
a dual-state sensor (102) coupled to the medical device (100), wherein the dual-state sensor (102) comprises two states:
a sterilized state indicative of the dual-state sensor (102) being exposed to a sterilization process; and
a used state indicative of the dual-state sensor (102) being exposed to an external stimulus defining that the medical device (100) has been used.

2. The medical apparatus as claimed in claim 1, wherein the external stimulus comprises one or more of:
an external mechanical force;
a current or voltage;
an electric field; and
a magnetic force.

3. The medical apparatus as claimed in any one of the preceding claims, wherein the dual-state sensor (102) comprises:
a switch (302) electrically connected to the medical device (100); and
an indicator capacitor (304) electrically connected to the switch (302).

4. The medical apparatus as claimed in claim 3, wherein the switch (302) comprises one or more of:
a temperature dependent mechanical switch;
a bimetal switch;
an electrical field dependent sensor; and
a temperature dependent resistance.

5. The medical apparatus as claimed in claim 1, wherein the dual-state sensor (102) comprises a shape memory alloy, SMA, temperature sensitive switch electrically connected to the medical device (100), wherein the SMA temperature sensitive switch is adapted to mechanically deform based on an external mechanical force and return to its original shape when exposed to a sterilization process.

6. The medical apparatus as claimed in claim 5, wherein the dual-state sensor (102) comprises:
a shape memory alloy, SMA, temperature sensitive switch adapted to return to its original shape when exposed to a sterilization process; and
a pin (506) mechanically connected to the SMA temperature sensitive switch, wherein the pin is adapted to deform the SMA temperature sensitive switch when the medical apparatus is in use.

7. The medical apparatus as claimed in any one of the preceding claims, wherein the dual-state sensor (102) further comprises an active electronic circuit (306) electrically connected to the dual-state sensor (102), wherein the active electronic circuit (306) is adapted to count the number of times the dual-state sensor (102) has been in a used state.

8. The medical apparatus as claimed in claim 1, wherein the dual-state sensor (102) comprises a material adapted to:
change color based on being exposed to a sterilization process; and
return to an original color based on being exposed to an external mechanical force.

9. A system for indicating whether a medical apparatus has been sterilized, the system comprising;
a medical apparatus as claimed in any one of the preceding claims;
a patient monitor (200) electrically coupled to the medical apparatus, wherein the patient monitor is configured to detect the state of the dual-state sensor (102).

10. The system as claimed in claim 9, wherein the patient monitor (200) is further configured to count how many times the dual-state sensor (102) has been in a used state.

11. A method for indicating whether a medical apparatus has been used or sterilized, the method comprising:
indicating the state of a dual-state sensor (102) on a medical device (100), wherein the dual-state sensor (102) comprises two states:
a sterilized state indicative of the dual-state sensor (102) being exposed to a sterilization process; and
a used state indicative of the dual-state sensor (102) being exposed to an external stimulus defining that the medical device (100) has been used.;
based on the dual-state sensor (102) being in the sterilized state, indicating that the medical device (100) has not been used; and
based on the dual-state sensor (102) being in the used state, indicating that medical device (100) has been sterilized.

12. The method for counting how many times a medical apparatus has been sterilized, the method comprising:
indicating whether a medical apparatus has been used or sterilized, using the method as claimed in claim 11; and
based on the medical apparatus having been sterilized, increasing a count by one, wherein the count indicates the number of times the medical apparatus has been sterilized.

13. The method as claimed in claim 12, further comprising:
displaying the count; and
displaying the state of the dual-state sensor (102).

14. The method as claimed in any one of claims 12 or 13, further comprising
determining a unique identifying number for a medical apparatus;
obtaining a count associated with the unique identifying number; and
based on the medical apparatus having been sterilized, increasing the count by one.

15. A computer program comprising code means for implementing the method of any one of claims 11 to 14 when said program is run on a processing system.
